# EUROPEAN PATENT SPECIFICATION

(11) **EP 1 175 511 B1**
(45) Date of publication and mention of the grant of the patent: **26.10.2005**
(21) Application number: 00917511.8
(22) Date of filing: 07.04.2000
(51) Int. Cl.: C12Q 1/68, B01L 3/00

(54) **METHOD AND KITSET FOR SAMPLING AND STORAGE OF BIOLOGICAL SAMPLES**
METHODEN UND TESTSÄTZE ZUR PROBENNAHME UND AUFBEWAHRUNG VON BIOLOGISCHEN PROBEN
PROCEDE ET TROUSSE POUR RECUEILLIR ET STOCKER DES PRELEVEMENTS BIOLOGIQUES

(30) Priority: 13.04.1999 NZ 33523399
(43) Date of publication of application: 30.01.2002
(73) Proprietor: Lingard Label Company Limited, Hornby, Christchurch (NZ); Agresearch Limited, Hamilton 2001 (NZ)
(72) Inventor: SHIRLEY, Kevin, John, Christchurch (NZ); TATE, Michael, Lewis, Dunedin (NZ)
(74) Representative: Goodwin, Mark
(86) International application number: PCT/NZ2000/000050
(87) International publication number: WO 2000/061802

(56) References cited:
- EP-A- 0 741 296
- FR-A- 2 621 690
- US-A- 2 734 503
- US-A- 5 092 466
- US-A- 5 496 562
- WATANABE GOTARO ET AL: "ABO blood grouping of old bloodstains by ELISA using adhesive tape." RESEARCH AND PRACTICE IN FORENSIC MEDICINE, vol. 37, 1994, pages 87-92, XP000978850 ISSN: 0289-0755

## Description

### Technical Field

This invention relates to a method and a kitset for collecting and storing a plurality of biological samples for later identification of individual samples. It is particularly suited for the collection and storage of sources of DNA, for example of blood. In a particularly preferred embodiment it may be used in a system for tracing meat specimens back to the carcass from which the meat originated.

### Background Art

US 5,092,466 describes a very sophisticated storage system for storing very large numbers of samples of protein gene products or hybridoma cells. The samples are collected on a porous medium such as filter paper. The samples are then lyophilised and placed in an air tight packet. This packet is then welded on to motion picture film stock with appropriate identification such as a bar code. The sample can be punched out of the film. The film can be spliced in the same manner as normal movie stock. This is a very convenient system for storing millions of samples in a retrievable compact storage system.

US 5,460,057 describes a method and apparatus particularly adapted for handling blood samples. This also involves the use of filter paper to collect the samples. The filter paper is attached to an identification label. When the samples are to be analysed a punch is used to punch out a small portion of the sample on the filter paper and it is then analysed.

A suitable filter paper medium for absorbing and storing of DNA samples is described in US Patent 5,496,562. This is a solid matrix impregnated with a compound or composition which protects against degradation of DNA which is incorporated into or absorbed on the matrix. The matrix claimed is a cellulose based matrix. The impregnated DNA stabilising composition consists of a weak base, a chelating agent, an ionic surfactant or detergent and optionally uric acid or a urate sale.

Other DNA storage matrices are described in US Patents 5,756,126; 5,807,527; 5,972,360; 5,976,572 and 5,985,327. Similar DNA storage matrices are described in WO 90/03959 and AU 691,114.

EP 0741 296A relates to a pre-transfusion card designed to determine the compatibility of donor and recipient blood.

US 2,734,503 describes a bandage comprising a polymeric elastic backing on which is superimposed an adherent pad dressing, one side of the backing being covered with an adhesive.

FR 2621690A relates to a device intended to act as a support for samples, in particular biological samples.

While systems are known from the prior art described above for the identification storage and retrieval of DNA samples under laboratory conditions. When samples are to be taken from large numbers of animals in a race or pen or moving carcasses on the killing chain of an animal slaughterhouse quick and accurate sample taking is required.
It an object of this invention to go some way towards achieving this desideratum or at least to offer the public a useful choice.

Although the present invention was conceived in the environment of a killing chain, its use is not limited thereto and can be used in any system where biological samples need to be taken from a plurality of individual sources, stored and then are able to be individually traced back to their original sources.

### Summary of Invention

Accordingly, the invention may be said broadly to consist in a method for collecting and storing a biological DNA sample for later identification of the source of said sample which comprises the steps of:
providing a sampling strip, one face of which has an absorbent area impregnated with a DNA stabilising composition, and an adhesive area; the other face of which covers and protects the sampling area,
collecting a biological DNA sample on said absorbent area,
providing an identification substrate having a surface area to receive said sampling strip, applying said sampling strip containing said biological DNA sample to said substrate so that said absorbent area is enveloped between the outer face of said strip and said substrate,
marking each said substrate and/or said sampling strip either before, during or after said applying stage with indicia to identify the source of the biological DNA sample collected on said sampling strip, and
storing said combined marked substrate/strip.

Preferably said biological DNA sample is blood, lymph or saliva.

Preferably said biological sample is taken from an animal carcass.

In one embodiment said identification substrate is a carcass ticket.

Alternatively said DNA sample is taken from a live animal.

In another embodiment said substrate is a document of description.

Preferably said method is used within a system to trace the origin of meat or other food stuffs.

Preferably said method is used within a system to trace the origin of meat or other food stuffs.

Preferably said marked combined substrate/strip is stored in dry storage.

Preferably said marking is done on said identification substrate before said sampling strip is affixed thereto.

In another embodiment the invention may be said broadly to consist in a biological DNA sample storage and identification kitset comprising:
a sampling strip consisting essentially of:
   a backing sheet, one side of which covers and protects the sampling area,
   a layer of adhesive covering and adhered to the other face of said backing sheet,
   an absorbent area impregnated with a DNA stabilising composition adhered to adhesive layer on said backing sheet, the area covered by said absorbing sheet being limited to leave a sufficient area of exposed adhesive to allow said backing sheet to be stuck to a substrate with said absorbing sheet therebetween, and
   a substrate to which said sampling strip may be affixed by said adhesive, said substrate being capable of receiving printed indicia to identify the source of said sample.

More preferably said sample is blood, lymph or saliva.

Preferably said adhesive is capable of maintaining adhesion when frozen.

Preferably said adhesive is a food grade adhesive.

Preferably said adsorbing sheet is DNA sampling paper.

Preferably said filter paper is treated to maintain DNA in a stable state.

Preferably said sampling strip is supplied with said adhesive and sampling sheet face down on a sheet of roll stock whose surface has low adhesivity to allow quick removal of said sampling strip.

Preferably there are perforations through said backing sheet along outer periphery of said sampling sheet.

In one embodiment said substrate is a carcass ticket.

In another embodiment said substrate is a document of description.

In an alternative embodiment a first portion of said sampling strip is adhered to or is integral with said substrate and a second portion faces away from said substrate, a release sheet being releasably adhered to said second portion of said sampling strip, said substrate being adapted to receive said second portion and to be adhered thereto when said release sheet has been removed and said second portion is applied thereto.

The invention may also be said to consist in a method as defined above which comprises using said alternative kitset embodiment, removing said release sheet, applying said sample absorbing sheet to said source of DNA and sticking said second portion of said sampling strip to said substrate.

### Brief Description of the Drawing

Figure 1 is a schematic diagram of the method according to the invention.
Figure 2 is a schematic diagram of how the method and kitset of the invention may be used in a process of tracing specimen DNA samples to their carcass source.
Figure 3 is the sectional view III-III in Figure 4.
Figure 4 is a top plan view of a sampling strip according to the invention on roll stock being dispensed from a roll dispenser.
Figure 5 is a top plan view of a carcass ticket.
Figure 6 is a plan view of an open page of an embodiment of a document of description, in this example an animal passport.

### Detailed Description of the Invention

### Sampling and Storage

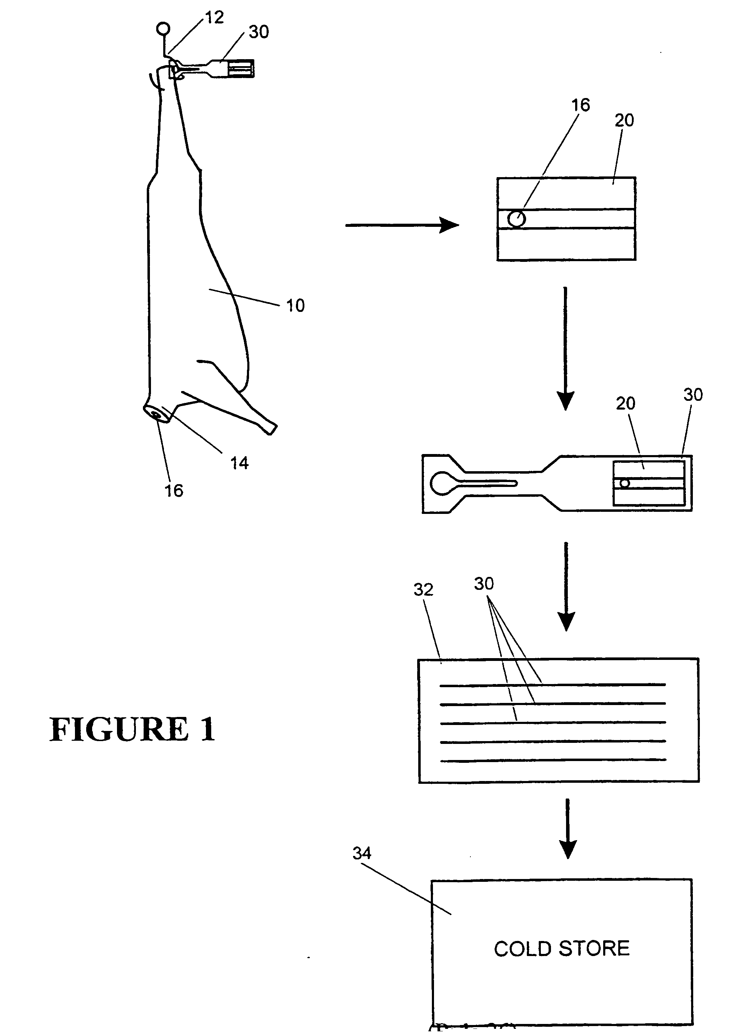

Illustrated in Figure 1 is an animal carcass 10 slung from a carrying hook 12 along a killing chain in an abattoir. Attached to the hook 12 is a carcass ticket 30 which will be described in more detail below. The carcass ticket 30 may also be attached to the leg of the carcass in a manner well known to those skilled in the art.

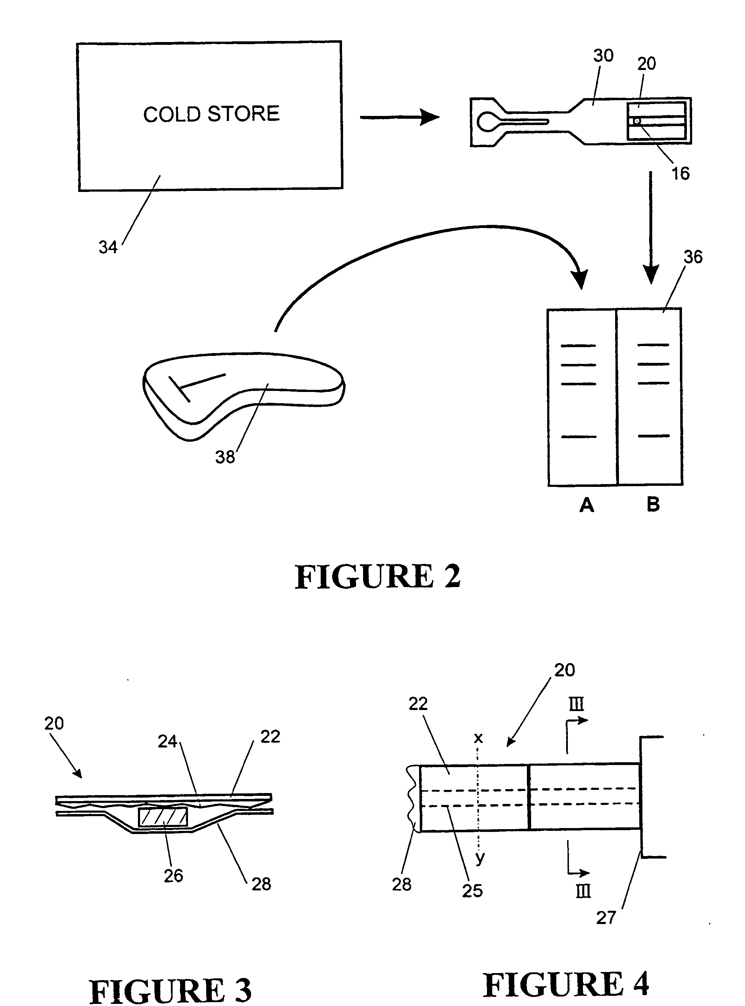

On the neck 14 of the carcass 10 is the blood or lymph sample 16 to be collected. An operator standing next to the chain will remove a sampling strip 20 from the roll stock 28 being dispensed from a dispenser 27 (described below with reference to Figure 4). The operator applies the sampling strip 20 face against the sample 16 so that the sample 16 is absorbed on the absorbent sheet 26 of the sampling strip 20. The operator then attaches the sampling strip 20 to the carcass ticket 30 and the carcass ticket 30 continues through processing where the carcass is cut into meat cuts packaged and distributed. The carcass tickets 30 with the samples attached are gathered in bunches and stored in ticket containers 32. For long term storage carcass tickets 10 with sampling strips 20 attached are allowed to dry and are then placed in water vapour permeable containers 32. These are stored in a dry environment either frozen (cold storage 34) or at room temperature.

At a point in the killing chain earlier than where the DNA sampling is done the carcass ticket 30 will have been labelled with identification indicia, such as bar coding, to identify the particular carcass 10.

A unique code could optionally be printed on the DNA-sampling strip 20 at the time of initial manufacture, or during delivery of the strip 20 via the dispenser 27.

### DNA Tracing

The purpose of storing DNA samples 16 with records of the individual sources of those samples on carcass tickets 30 is to allow tracing from meat cuts. Referring to Figure 2, when a positive identification with the source needs to be made from a meat cut 38 a specimen is taken from the cut and subjected to genetic analysis. At the same time the carcass ticket storing the DNA sample from the suspected source of the meat specimen is also subjected to a DNA analysis on gels 36. The electrophoretic gels A and B illustrated here show that there is a match between the meat cut 38 and the carcass 10.

### Sampling Strip

The structure of the sampling strip is illustrated in Figures 3 and 4. In a preferred embodiment the backing sheet 22 is a clear polystyrene film sold by Dow Chemical Company under the trade name "Opticite". The thickness is 0.076 mm. This product is compliant with the regulations of the US Food and Drug Administration (FPA) and thus may come into contact with meat carcasses intended for human consumption.

The adhesive 24 attached to backing strip 22 is "550" adhesive sold by Green Bay Packaging Inc of Green Bay, Wisconsin, USA. It is intended for use in a cold environment. It may be applied at temperatures above minus 15°C. The minimum temperature for retaining adhesiveness is minus 49°C.

The absorbent sheet 26 on sample collecting strip 20 is preferably selected from two types of absorbent paper:
a) natural cellulose impregnated as described above in relation to US 5,496,562. This is sold as "FTA" paper by Whatman PLC, and
b) uncoated cotton linter paper produced by Schleicher and Schuell Inc under the brand name "SNS 903".

Both types of paper are suitable for direct contact with fatty and aqueous food. The latter product is a class 11 medical device listed by the FDA. The weight range is 185 to 210 gsm.

As illustrated in Figures 3 and 4, the sampling strip 20 is adhered to the release side of roll stock 28 which is despatched out of a dispenser 27. The dispenser is preferably one which retains the roll stock while a strip is being lifted and then releases a further length of roll stock with a sampling strip 20 attached.

The roll stock is a 70 gsm densified kraft sheet commonly used in the self adhesive industry. It has a soventless silicon release coating on the face to which the sampling strip 20 is attached so as to release the strip as required.

There are a series of perforations 25 through backing sheet 22. These are at either edge of absorbent sheet 26.

In a preferred embodiment absorbent sheet 26 has an orientation. One end is for handling and the other is where the sample 16 is collected as shown. The orientation can be indicated by making the backing sheet 22 transparent at the sample end and translucent or opaque at the handling end.

Another alternative manner of using a sampling strip 20 will be described with reference to figure 4. In this alternative the sampling strip 20 is folded along the line XY. The roll stock 28 is peeled from the right end to the line XY and the exposed portion applied to the carcass ticket 30. The remaining half is folded back with the piece of roll stock 28 remaining in place. When a carcass is to be sampled the roll stock 28 is removed and the sample taken by an operator holding the carcass ticket 30. The portion of the sampling strip 20 to the left of line XY, with a sample 16 taken, is then folded back to be attached to carcass ticket 30.

In a further alternative the portion of the sampling strip 20 to the right of line X-Y is integral with a substrate. In such an embodiment the absorbent sheet 26 and the adhesive layer 24 would not be present on the portion of the backing sheet 22 which is integral with substrate 28.

When a genetic analysis is to be made of a sample 16 from a sampling strip 20 as described with reference to Figure 2 the usual procedure is to place the carcass ticket 30 with the sampling strip 20 attached to it in a punch and punch out the sample 16 on which the analysis is done. As an alternative to this it is possible to remove the absorbing sheet in its entirety by pulling it loose along the perforations 25.

### Carcass Ticket

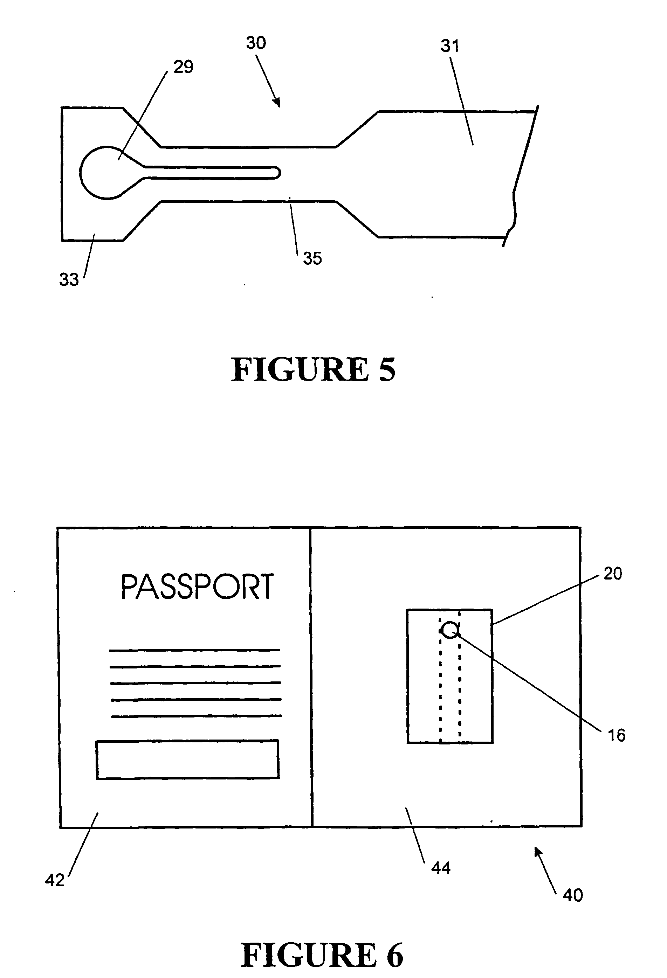

A carcass ticket 30 is illustrated in Figure 5. It consists if a head portion 33, a neck portion 35 and a body portion 31. A hole 29 is cut through the head and neck portion to allow the tag to be hooked on to the hook from which the carcass is to be hung or onto the leg of the carcass itself. The body portion 31 serves to receive the sampling strip and also to be printed with the information identifying the source of the carcass 10.

In a preferred embodiment the carcass ticket 30 is a white polypropylene base sheet coated on one side with a thermal imaging coating with which to produce the identification information. The thermal coating is protected with an additional environmental protection coating to provide oil, water, heat, light and plasticiser resistance to the thermal coating. The imaging is black.

### Documents of Description

Another use of the method of the invention is to provide positive identification on a document describing one or more animals. Such documents are referred to in this specification as "documents of description". A document of description may be a single page, a folder or a book. It will contain a written number or letter identifier and a sample strip with a sample attached to a page of the document, the page being a substrate. Common examples of where a document of description is used include: animal registration or licensing systems, in animal health control programmes, bills of sale, entry form for a contest; during movement or import and export of animals or products from animals (animal passport). If there is any doubt as to the documentation then a DNA sample is taken from the animal and compared to the DNA sample in the document of description produced in relation to that animal.

Illustrated in Figure 6 is a document of description in the form of an animal passport 40. The cover page 42 has the usual information. The sample page 44 serves as substrate function similar to that of the carcass ticket in the embodiment illustrated in Figures 1-5. The sampling strip 20 with the DNA sample 16 is stuck to the sample page 44 of the passport. It can be made of material similar to that of the carcass ticket 30.

At the time that the passport is being issued a blood sample may be taken on the sampling strip 20 which is then attached to the passport sample page 44 for the permanent record. The page may be laminated with a clear plastics material for additional security and for a longer storage life.

### Example 1: Collection, Storage and Tracing of DNA Sample from Carcass

Samples were collected by wiping a volume of blood from the neck of a carcass onto a DNA-sampling strip 20. The DNA-sampling strip was then attached by permanent adhesive 24 to the carcass identification ticket 30 hanging from the carcass 10. This procedure was used to sample all carcasses processed for a 10 metric tonne consignment of meat requiring traceability. The carcass identification tickets 30 with DNA sampling strips 20 attached were stored in cardboard boxes containing approximately 1000 tickets in numbered bunches of approximately 60 tickets. The tickets in each bunch were tied securely in the order of cutting. The boxes of DNA samples were entered onto an inventory system and stored at -20°C. DNA analysis of these stored tickets samples was used to trace meat from the market.

A trace was requested on 3 portions of meat from the market. Samples of the meat were taken by slicing open the meat and placing an FTA® Card (Flinders Technologies PTY Ltd) into the cut, then closing the cut tightly until the FTA Card was saturated. The FTA Card was then air dried and mailed to a laboratory for analysis. Once received, a 1.2mm diameter punch of each bloodstain was removed using a HARRIS MICRO-PUNCH™ (Shunderson Communications) and placed into a separate bar-coded or otherwise uniquely identified tube or receptacle. The identification of the receptacle and of the meat sample were entered into a spreadsheet or database. These data were used at a later date to relate the DNA profile to the sample identification. Both the FTA Card and Harris MICRO-PUNCH were obtained from the distributor Life Technologies, Inc., Gathersburg, MD, USA.

The date and time of production information on packaging of the meat was supplied to the ticket storage facility. The appropriate box of stored carcass tickets was identified and sent to the laboratory. A 1.2mm diameter punch from the blood stain attached to each relevant ticket was removed using a HARRIS MICRO-PUNCH™ (Shunderson Communications) and placed into a separate bar-coded (or otherwise uniquely identified) tube or receptacle. The identification of the receptacle and of the carcass ticket were entered into a spreadsheet or database and stored for later use in relating the DNA profile to the carcass information.

### Example 2: DNA analysis

Punches from FTA® card and from the sampling strip 20 were each washed as follows. Using a Biomek 2000 Automated Workstation (Beckman Instruments, USA), 160µl aliquots of FTA™ PURIFICATION REAGENT (Flinders Technologies PTY Ltd, supplied by Life Technologies Gibco-BRL) were dispensed into each well. Reagent was allowed to sit for 5 min at room temperature. After the 5 min incubation the reagent not soaked into the punch was removed. The above steps were repeated an additional two times for a total of three washes with the FTA™ Purification Reagent. After the FTA™ Purification Reagent has been removed for the third time, 160µl of TE (10 mM Tris-HCl, pH 8.0, 0.1mM EDTA) was dispersed into each well and mixed. The mixtures were allowed to sit for 5 min at room temperature. After the 5 min incubation most of the TE was aspirated. An additional 160µl of TE buffer was added. After the final TE buffer had been removed, 160µl 96% ethanol was added, incubated for 5 min and aspirated.

The FTA paper punch was allowed to air dry completely (approximately 2 hours at room temperature). Alternatively, drying was accelerated by incubating the plate of punches at 60°C for 30 min.

The washed punches were analysed by the polymerase chain reaction.

The resulting samples from the meat specimen punch and the carcass sample punch were loaded onto separate lanes of ABI 377 gel (36cm plate); Run module GS 36C/2400 (Applied Biosystems Inc.).

The output of ABI377 was analysed using Genescan and Genotyper Software (Applied Biosystems Inc.). The results from each carcass ticket sample were compared with the profile from the meat sample. Profiles identical within the tolerance of the analysis system (plus or minus lbp) are selected as "matches". The likelihood that this match could have occurred by chance was calculated using standard methods and allele frequency data derived from the other animals analysed at the time. A single matching carcass ticket sample was identified for each meat sample. In each case the likelihood that such a DNA match could occur with a carcass ticket from a different animal was less than one chance in one million tests.

The DNA link between the sample of meat from the market place and the stored carcass sample was used relate information previously only associated the carcass to the submitter of the sample of the meat cut. The data included farm of origin, farm and transport quality assurance data, weight and grade of carcass.

### Other Applications

Although the method of the invention has been described in relation to DNA sampling, other potential analyses which could be done on the samples taken using the DNA sampling strip include:
1) Qualitative or quantitative analysis of microbes or products produced by microbes,
2) Genotyping or DNA sequencing from DNA or RNA for example for the purposes of animal identification, parentage testing, species or strain identification, or testing for the presence or absence of a specific gene or mutation, and
3) Quantitative or qualitative analysis of proteins.

The method and kitset of the invention can be used in any application requiring rapid sampling of fluid from plant or animal tissue for diagnostic or reference purposes and clear identification of the sample is paramount.

Possible applications including collecting samples from live animals including humans, dead animals/humans or tissue. Samples would include blood, saliva, mucus, plasma, body fluid such as milk and crushed or homogenised tissue such as muscle. The sampling strip with the sample is stuck to pre-existing, or custom printed document of description concerning the animal or human (including a book, passport, photograph, card, tag, ticket - any format for printed material).

For example, when animals are being tagged or handled a sample of blood is collected by soaking one or two drops of blood onto the DNA sampling strip 20 label. The sampling strip is then permanently stuck to a substrate 30 in the form of a card, book or passport, or photograph which lists all details concerning the animal tag (eg: colour, number, radio or other electromagnetic frequency emission). This information could be pre-existing or could be printed just prior to or during the sample being taken.

The method and kitset of this invention could also be used in the same way to trace the plant origin of materials. The plant products to be traced could be sap, fruit juice, or crushed or homogenised plant tissue.

Other variations and permutations within the scope of the claims will be apparent to those skilled in the art.

## Claims

1. A method for collecting and storing a biological DNA sample for later identification of the sources of said sample which comprises the steps of:
providing a sampling strip, one face of which has an absorbent area impregnated with a DNA stabilising composition, and an adhesive area; the other face of which covers and protects the sampling area,
collecting a biological DNA sample on said absorbent area,
providing an identification substrate having a surface area to receive said sampling strip,
applying said sampling strip containing said biological DNA sample to said substrate so that said absorbent area is enveloped between the outer face of said strip and said substrate,
marking said substrate and/or said sampling strip either before, during or after said applying stage with indicia to identify the source of the biological DNA sample collected on said sample collecting strip, and
storing said combined marked substrate/strip.

2. A method as claimed in claim 1 wherein the sampling strip has perforations through said sampling strip, the perforations only being situated along the outer periphery of said sampling area.

3. A method as claimed in any one of claims 1 to 2 wherein said biological DNA sample is blood, lymph or saliva.

4. A method as claimed in any one of claims 1 to 3 wherein said biological sample is taken from an animal carcass.

5. A method as claimed in any one of claims 1 to 4 wherein said identification substrate is a carcass ticket.

6. A method as claimed in any one of claims 1 to 3 wherein said biological sample is from a live animal.

7. A method as claimed in any one of claims 1 to 3 and 6 wherein said substrate is a document of description.

8. A method as claimed in any one of claims 1 to 5 wherein said method is used within a system to trace the origin of meat or other foodstuffs.

9. A method as claimed in any one of claims 1 to 5 and 8 wherein said marked combined substrate/strip is stored in dry storage.

10. A method as claimed in any one of claims 1 to 5, 8 and 9 wherein said marking is done on said identification substrate before said sampling strip is affixed thereto.

11. A biological DNA sample storage and identification kitset comprising:
a sampling strip consisting essentially of:
a backing sheet, one side of which covers and protects the sampling area,
a layer of adhesive covering and adhered to the other face of said backing sheet,
an absorbent area impregnated with a DNA stabilising composition adhered to said adhesive layer on said backing sheet, the area covered by said absorbing area being limited to leave a sufficient area of exposed adhesive to allow said backing sheet to be stuck to a substrate with said absorbent area therebetween, and
a substrate to which said sampling strip may be affixed by said adhesive, said substrate being capable of receiving printed indicia to identify the source of said sample.

12. A kitset as claimed in claim 11 wherein the backing sheet has perforations through said backing sheet, the perforations only being situated along the outer periphery of said sampling area.

13. A kitset as claimed in any one of claims 11 to 12 wherein said sample is blood, lymph or saliva.

14. A kitset as claimed in any one of claims 11 to 13 wherein said adhesive is capable of maintaining adhesion when frozen.

15. A kitset as claimed in any one of claims 11 to 14 wherein said adhesive is a food grade adhesive.

16. A kitset as claimed in any one of claims 11 to 15 wherein said sampling strip is supplied with said adhesive and sampling sheet face down on a sheet of roll stock surface has low adhesivity to allow quick removal of said sampling strip.

17. A kitset as claimed in any one of claims 11 to 16 wherein said substrate is a carcass ticket.

18. A kitset as claimed in any one of claims 11 to 16 wherein said substrate is a page of a document of description.

19. A kitset as claimed in any one of claims 11 to 15 and 17 to 18 wherein a first portion of said sampling strip is adhered to or is integral with said substrate and a second portion faces away from said substrate, a release sheet being releasably adhered to said second portion of said sampling strip, said substrate being adapted to receive said second portion and to be adhered thereto when said release sheet has been removed and said second portion is applied thereto.

20. A method according to any one of claims 1 to 10 which comprises using a kitset according to claim 19, removing said release sheet, applying said sample absorbing sheet to said source of DNA and sticking said second portion of said sampling strip to said substrate.

21. A DNA sampling strip which has one face including
an absorbent area, and
an adhesive area,
the sampling strip **characterised in that** the absorbent area includes DNA sampling paper impregnated with a DNA stabilising composition, and the strip is further **characterised in that** the other face of the strip covers and protects the sampling area.

22. A sampling strip as claimed in claim 21, wherein the sampling strip has perforations through said sampling strip, the perforations only being situated along the outer periphery of said sampling area.

## Patentansprüche

1. Verfahren zum Entnehmen und Aufbewahren einer biologischen DNA-Probe zum späteren Nachweis der Quellen der Probe mit den Schritten:
Bereitstellen eines Probenstreifens, wovon eine Fläche einen Absorptionsbereich, der mit einer DNA-stabilisierenden Zusammensetzung getränkt ist, und einen Adhäsionsbereich aufweist, und die andere Fläche den Probenbereich bedeckt und schützt,
Aufnehmen einer biologischen DNA-Probe auf dem Absorptionsbereich,
Bereitstellen eines Nachweissubstrates mit einem Oberflächenbereich zum Aufnehmen des Probenstreifens, Auflegen des Probenstreifens, der die biologische DNA-Probe enthält, auf das Substrat, so dass der Absorptionsbereich zwischen der Außenfläche des Streifens und des Substrates eingehüllt wird,
Kennzeichnen des Substrates und/oder des Probenstreifens mit Kennzeichen entweder vor, während oder nach der Stufe des Auflegens, um die Quelle der biologischen DNA-Probe, die auf dem Probenentnahmestreifen aufgenommen ist, zu identifizieren, und
Aufbewahren des verbundenen **gekennzeichnet**en Substrates/Streifens.

2. Verfahren nach Anspruch 1,
wobei der Probenstreifen Bohrungen durch den Probenstreifen aufweist, und die Bohrungen sich nur entlang des Außenrandes des Probenbereiches befinden.

3. Verfahren nach einem der Ansprüche 1 bis 2,
wobei die biologische DNA-Probe Blut, Lymphe oder Speichel ist.

4. Verfahren nach einem der Ansprüche 1 bis 3,
wobei die biologische Probe von einem Tierkadaver entnommen ist.

5. Verfahren nach einem der Ansprüche 1 bis 4,
wobei das Nachweissubstrat ein Kadaveretikett ist.

6. Verfahren nach einem der Ansprüche 1 bis 3,
wobei die biologische Probe von einem lebenden Tier stammt.

7. Verfahren nach einem der Ansprüche 1 bis 3 und 6,
wobei das Substrat ein Beschreibungsdokument ist.

8. Verfahren nach einem der Ansprüche 1 bis 5,
wobei das Verfahren in einem System zum Aufspüren der Herkunft von Fleisch oder anderen Nahrungsmitteln verwendet wird.

9. Verfahren nach einem der Ansprüche 1 bis 5 und 8,
wobei der **gekennzeichnet**e verbundene Substrat/Streifen in trockener Lagerung aufbewahrt wird.

10. Verfahren nach einem der Ansprüche 1 bis 5, 8 und 9,
wobei die Kennzeichnung auf dem Nachweissubstrat erfolgt, bevor der Probenstreifen daran befestigt wird.

11. Testsatz zur Aufbewahrung und zum Nachweis biologischer DNA-Proben mit:
einem Probenstreifen, der im wesentlichen besteht aus:
einem Deckblatt, wovon eine Seite den Probenbereich bedeckt und schützt,
einer Klebstoffschicht, die die andere Fläche des Deckblattes bedeckt und daran angeklebt ist,
einem Absorptionsbereich, der mit einer DNA-stabilisierenden Zusammensetzung getränkt ist und an die Adhäsionsschicht auf dem Deckblatt angeklebt ist, wobei der Bereich, der durch den Absorptionsbereich bedeckt wird, begrenzt ist, um eine ausreichende Fläche an exponiertem Klebstoff freizulassen, damit das Deckblatt an das Substrat mit dem Absorptionsbereich dazwischengeklebt werden kann, und
einem Substrat, an das der Probenstreifen mittels des Klebstoffes befestigt werden kann, und
das Substrat in der Lage ist, gedruckte Kennzeichen aufzunehmen, um die Quelle der Probe zu identifizeren.

12. Testsatz nach Anspruch 11,
wobei das Deckblatt Bohrungen durch das Deckblatt aufweist, und die Bohrungen sich nur entlang des Außenrandes des Probenbereiches befinden.

13. Testsatz nach einem der Ansprüche 11 bis 12,
wobei die Probe Blut, Lymphe oder Speichel ist.

14. Testsatz nach einem der Ansprüche 11 bis 13,
wobei der Klebstoff in der Lage ist, auch gefroren die Anhaftung beizubehalten.

15. Testsatz nach einem der Ansprüche 11 bis 14,
wobei der Klebstoff ein lebensmittelunbedenklicher Klebstoff ist.

16. Testsatz nach einem der Ansprüche 11 bis 15,
wobei der Probenstreifen mit dem Klebstoff versehen ist, und das Probenblatt mit der Fläche nach unten auf einem Blatt einer Vorratsrolle angeordnet ist, dessen Oberfläche ein geringes Haftvermögen aufweist, um ein rasches Entfernen des Probenstreifens zu ermöglichen.

17. Testsatz nach einem der Ansprüche 11 bis 16,
wobei das Substrat ein Kadaveretikett ist.

18. Testsatz nach einem der Ansprüche 11 bis 16,
wobei das Substrat eine Seite eines Beschreibungsdokumentes ist.

19. Testsatz nach einem der Ansprüche 11 bis 15 und 17 bis 18,
wobei ein erster Teil des Probenstreifens an das Substrat angeklebt oder damit integral ist, und ein zweiter Teil von dem Substrat abgewandt ist, einem Ablöseblatt, das lösbar an den zweiten Teil des Probenstreifens angeklebt ist, und das Substrat angepasst ist, den zweiten Teil aufzunehmen und daran angeklebt zu werden, wenn das Ablöseblatt entfernt worden ist und der zweite Teil darauf gelegt wird.

20. Verfahren nach einem der Ansprüche 1 bis 10 mit Verwenden eines Testsatzes nach Anspruch 19, Entfernen des Ablöseblattes, Auflegen des Probe absorbierenden Blattes auf die DNA-Quelle und Ankleben des zweiten Teils des Probenstreifens an das Substrat.

21. DNA-Probenstreifen, der eine Fläche aufweist, welche einen Absorptionsbereich und einen Adhäsionsbereich enthält,
wobei der Probenstreifen **dadurch gekennzeichnet ist, dass** der Absorptionsbereich DNA-Probenpapier enthält, das mit einer DNA-stabilisierenden Zusammensetzung getränkt ist, und der Streifen ferner **dadurch gekennzeichnet ist, dass** die andere Fläche des Streifens den Probenbereich bedeckt und schützt.

22. Probenstreifen nach Anspruch 21,
wobei der Probenstreifen Bohrungen durch den Probestreifen aufweist, und sich die Bohrungen nur entlang des Außenrandes des Probenbereiches befinden.

## Revendications

1. Procédé de collecte et de stockage d'un échantillon d'ADN biologique pour l'identification ultérieure des sources dudit échantillon qui comprend les étapes de :
fournir une bandelette d'échantillonnage, dont une face a une zone absorbante imprégnée d'une composition stabilisant l'ADN, et une zone adhésive ; dont l'autre face couvre et protège la zone d'échantillonnage,
collecter un échantillon d'ADN biologique sur ladite zone absorbante,
fournir un substrat d'identification ayant une surface pour recevoir ladite bandelette d'échantillonnage,
appliquer ladite bandelette d'échantillonnage contenant ledit échantillon d'ADN biologique audit substrat de sorte que ladite zone absorbante est enveloppée entre la face externe de ladite bandelette et ledit substrat,
marquer ledit substrat et/ou ladite bandelette d'échantillonnage avant, pendant ou après ladite étape d'application avec des indices pour identifier la source de l'échantillon d'ADN biologique collecté sur ladite bandelette de collecte d'échantillon, et
stocker ledit substrat/bandelette combiné marqué.

2. Procédé selon la revendication 1, dans lequel la bandelette d'échantillonnage a des perforations à travers ladite bandelette d'échantillonnage, les perforations n'étant situées que le long de la périphérie externe de ladite zone d'échantillonnage.

3. Procédé selon l'une quelconque des revendications 1 à 2, dans lequel ledit échantillon d'ADN biologique est le sang, la lymphe ou la salive.

4. Procédé selon l'une quelconque des revendications 1 à 3, dans lequel ledit échantillon biologique est prélevé d'une carcasse animale.

5. Procédé selon l'une quelconque des revendications 1 à 4, dans lequel ledit substrat d'identification est une étiquette de carcasse.

6. Procédé selon l'une quelconque des revendications 1 à 3, dans lequel ledit échantillon biologique provient d'un animal vivant.

7. Procédé selon l'une quelconque des revendications 1 à 3 et 6, dans lequel ledit substrat est un document de description.

8. Procédé selon l'une quelconque des revendications 1 à 5, dans lequel ledit procédé est utilisé dans un système pour retrouver l'origine de la viande ou autre denrée alimentaire.

9. Procédé selon l'une quelconque des revendications 1 à 5 et 8, dans lequel ledit substrat/bandelette marqué combiné est stocké en stockage à sec.

10. Procédé selon l'une quelconque des revendications 1 à 5, 8 et 9, dans lequel ledit marquage est effectué sur ledit substrat d'identification avant que ladite bandelette d'échantillonnage n'y soit fixée.

11. Trousse de stockage et d'identification d'un échantillon d'ADN biologique comprenant :
une bandelette d'échantillonnage constituée essentiellement par :
une feuille support, dont un côté couvre et protège la zone d'échantillonnage,
une couche d'adhésif couvrant et collée à l'autre face de ladite feuille support,
une zone absorbante imprégnée d'une composition stabilisant l'ADN collée à ladite couche adhésive sur ladite feuille support, la zone couverte par ladite zone absorbante étant limitée pour laisser une zone suffisante d'adhésif exposé pour permettre à ladite feuille support d'être collée à un substrat avec ladite zone absorbante entre les deux,
et
un substrat auquel ladite bandelette d'échantillonnage peut être fixée par ledit adhésif, ledit substrat étant capable de recevoir des indices imprimés pour identifier la source dudit échantillon.

12. Trousse selon la revendication 11, dans laquelle la feuille support a des perforations à travers ladite feuille support, les perforations n'étant situées que le long de la périphérie externe de ladite zone d'échantillonnage.

13. Trousse selon l'une quelconque des revendications 11 à 12, dans laquelle ledit échantillon est le sang, la lymphe ou la salive.

14. Trousse selon l'une quelconque des revendications 11 à 13, dans laquelle ledit adhésif est capable de maintenir l'adhésion quand il est congelé.

15. Trousse selon l'une quelconque des revendications 11 à 14, dans laquelle ledit adhésif est un adhésif de qualité alimentaire.

16. Trousse selon l'une quelconque des revendications 11 à 15, dans laquelle ladite bandelette d'échantillonnage est fournie avec ledit adhésif et la feuille d'échantillonnage avec une face vers le bas sur une feuille de surface de papier régide a une faible adhésivité pour permettre le retrait rapide de ladite bandelette d'échantillonnage.

17. Trousse selon l'une quelconque des revendications 11 à 16, dans laquelle ledit substrat est une étiquette de carcasse.

18. Trousse selon l'une quelconque des revendications 11 à 16, dans laquelle ledit substrat est une page d'un document de description.

19. Trousse selon l'une quelconque des revendications 11 à 15 et 17 à 18 dans laquelle une première partie de ladite bandelette d'échantillonnage est collée à ou intégrée avec ledit substrat et une seconde partie est tournée à distance dudit substrat, une feuille détachable étant collée de manière détachable à ladite seconde partie de ladite bandelette d'échantillonnage, ledit substrat étant adapté pour recevoir ladite seconde partie et y être collé, quand ladite feuille détachable a été retirée et que ladite seconde partie y est appliquée.

20. Procédé selon l'une quelconque des revendications 1 à 10, qui comprend l'utilisation d'une trousse selon la revendication 19, le retrait de ladite feuille détachable, l'application de ladite feuille absorbant l'échantillon à ladite source d'ADN et le collage de ladite seconde partie de ladite bandelette d'échantillonnage audit substrat.

21. Bandelette d'échantillonnage d'ADN qui a une face comprenant
une zone absorbante, et
une zone adhésive,
la bandelette d'échantillonnage **caractérisée en ce que** la zone absorbante comprend un papier d'échantillonnage d'ADN imprégné d'une composition stabilisant l'ADN, et la bandelette est en outre **caractérisée en ce que** l'autre face de la bandelette couvre et protège la zone d'échantillonnage.

22. Bandelette d'échantillonnage selon la revendication 21, dans laquelle la bandelette d'échantillonnage a des perforations à travers ladite bandelette d'échantillonnage, les perforations n'étant situées que le long de la périphérie externe de ladite zone d'échantillonnage.
